# FASCICULE DE BREVET EUROPEEN

(11) **EP 2 878 284 B1**
(45) Date de publication et mention de la délivrance du brevet: **08.01.2020**
(21) Numéro de dépôt: 14195333.1
(22) Date de dépôt: 28.11.2014
(51) Int. Cl.: A61F 5/02

(54) **Corset rigide asymétrique à valves latérales**
Asymmetrisches steifes Korsett mit seitlichen Ventilen
Asymmetric rigid corset with side valves

(30) Priorité: 02.12.2013 FR 1361952; 15.05.2014 FR 1454316
(43) Date de publication de la demande: 03.06.2015
(73) Titulaire: Lecante, 69008 Lyon (FR)
(72) Inventeur: Lecante, Cyril, 69160 Tassin la Demi Lune (FR); Pourret, Sophie, 69300 Caluire et Cuire (FR); De Mauroy de Curiere de Castelnau, Jean Claude, 38110 Saint Didier de la Tour (FR)
(74) Mandataire: Godard, Xavier

(56) Documents cités:
- EP-A1- 1 576 940
- FR-A1- 2 585 238
- FR-A1- 2 659 547
- FR-A1- 2 968 540

## Description

La présente invention est relative aux orthèses ou corsets de réduction de la scoliose et leurs variantes destinées à la cyphose thoracique et plus particulièrement aux corsets rigides asymétriques à valves latérales pour la réduction de la scoliose.

Elle est également relative à un procédé pour la conception et la réalisation du corset de chaque patient selon une méthode à la fois individualisée et standardisée.

On connait de nombreux corsets qui ont été décrits dans le cadre du traitement orthopédique non chirurgical de la scoliose.

On connait, par exemple, des corsets multi valves segmentaires constitués de plusieurs coquilles latérales fixées sur des montants antérieur et postérieur par l'intermédiaire de moyens de liaison permettant de régler le positionnement desdites coquilles latérales.

On connait également, des corsets pour la réduction tridimensionnelle des scolioses qui sont constitués de deux coquilles pelviennes et des coquilles lombaires et thoraciques reliées par des mats latéraux rigides, des sangles souples ventrales et une lame dynamique à ressort.

FR 2 659 547 décrit un corset orthopédique comprenant en combinaison: une coque moulée en une seule pièce formant la ceinture pelvienne et prenant appui sur les crêtes iliaques entourant le bassin au moyen de deux parties déformable élastiquement, de deux éléments souples et élastiques en toile provoquant une compression respectivement au niveau lombaire et thoracique, un élément auxiliaire d'appui destiné à enserrer l'aisselle de manière à rehausser l'une des épaules du patient, et deux mats métalliques antérieur et postérieur montés sur la ceinture pelvienne et permettant la fixation entre eux des éléments ci-dessus constituant le corset.

FR 2 585 238 divulgue un corset orthopédique modulaire tout plastique, thermoformable, transparent aux rayons x, comportant des mâts rigides, plusieurs coques pelviennes ou thoraco-pelvienne, des éléments de liaison tels que des barrettes à glissière et des éléments de réglages.

FR 2 968 540 divulgue un corset pour le maintien et la correction de la colonne vertébrale comportant un anneau pelvien bande à peu près circulaire une plaque abdominale deux coquilles latérales droite et gauche englobantes une platine de stabilisation postérieure.

Une difficulté connue pour la réalisation des corsets antérieurs réside dans la nécessité de disposer d'un moulage électronique en détorsion de la scoliose avec correction individualisée de la ou des courbures.

La présente invention a ainsi pour objet un corset rigide en polycarbonate à valves latérales asymétrique, reproduisant une colonne torse inverse de la scoliose et réalisé à l'aide d'un procédé de fabrication individualisé et standardisé.

La présente invention est définie par les revendications indépendantes 1 et 8. Des formes préférées de réalisation sont définies dans les revendications dépendantes.

Le procédé spécifique de conception et fabrication comporte une étape de positionnement du patient guidé par les mains du professionnel de santé, contrôlé en vidéo de face et de profil. Un moulage électronique est réalisé à l'issue de cette phase, avec les caractéristiques suivantes :
- Positionnement des pieds sur empreintes prédéfinies fixant l'axe vertical et le plan frontal
- Membres inférieurs tendus et bassin fixe.
- Eventuellement, disposition de deux supports verticaux que l'on peut déplacer qui peuvent servir de support aux membres supérieurs.
- Monitoring vidéo de face et de profil pour le positionnement du patient
- Utilisation d'un logiciel permettant la prise de vue en full 3D en moins de 3 secondes. Les positions demandées au patient sont en effet des positions extrêmes qui ne peuvent être maintenues que quelques secondes et non des postures stables.

Pour les besoins de la description, on retient l'exemple d"une scoliose à double courbure thoracique et lombaire, sans que cet exemple soit limitatif du domaine d'application de l'invention

Le corset rigide asymétrique à valves latérales pour la correction de la scoliose suivant la présente invention comprend au moins deux hémivalves latérales asymétriques, reproduisant une colonne torse en sens inverse de la scoliose, et au moins un montant (ou mat) postérieur, galbé en cypho-lordose, relié par des charnières postérieures aux hémivalves latérales, et au moins trois fermetures antérieures.

Le corset rigide asymétrique à valves latérales pour la correction de la scoliose suivant la présente invention comprend également un socle pelvien qui est symétrique et de forme quadrangulaire pour favoriser la stabilité sans compression abdominale antérieure ou sus-pubienne.

Le corset rigide asymétrique à valves latérales pour la correction de la scoliose suivant la présente invention comprend encore des charnières postérieures qui sont réglables dans le plan frontal.

Le corset rigide asymétrique à valves latérales pour la correction de la scoliose suivant la présente invention comprend aussi des fermetures antérieures constituées par une sangle velcro pour la fermeture supérieure et des sangles rigides à cliquets pour les deux fermetures inférieures.

Le procédé de conception et fabrication pour la réalisation du corset rigide asymétrique à valves latérales pour la correction de la scoliose suivant la présente invention consiste en :
I. un premier moulage électronique du tronc du patient en auto-élongation axiale active,
II. un second moulage électronique du tronc du patient en translation lombaire et lordose physiologique,
III. un troisième moulage électronique du tronc du patient en inflexion thoracique frontale et cyphose physiologique,
IV. la superposition des trois moulages électroniques pour reconstituer la colonne torse inverse de la scoliose du patient,
V. la réalisation du positif du gabarit par fraiseuse numérique 3D,
VI. le thermoformage de deux coques hémivalves droite et gauche en polycarbonate en un seul bloc sur le positif lissé.

La description qui va suivre et les dessins annexés, donnés à titre d'exemples non limitatifs, permettront de mieux comprendre l'invention, les caractéristiques qu'elle présente et les avantages qu'elle est susceptible de procurer.
Figure 1 est une vue de face avant représentant un corset rigide asymétrique à valves latérales pour la correction de la scoliose suivant la présente invention.
Figure 2 est une vue de coté montrant du corset rigide asymétrique à valves latérales pour la correction de la scoliose suivant la présente invention.
Figure 3 est une vue de face arrière ou de dos du corset rigide asymétrique à valves latérales pour la correction de la scoliose suivant la présente invention.

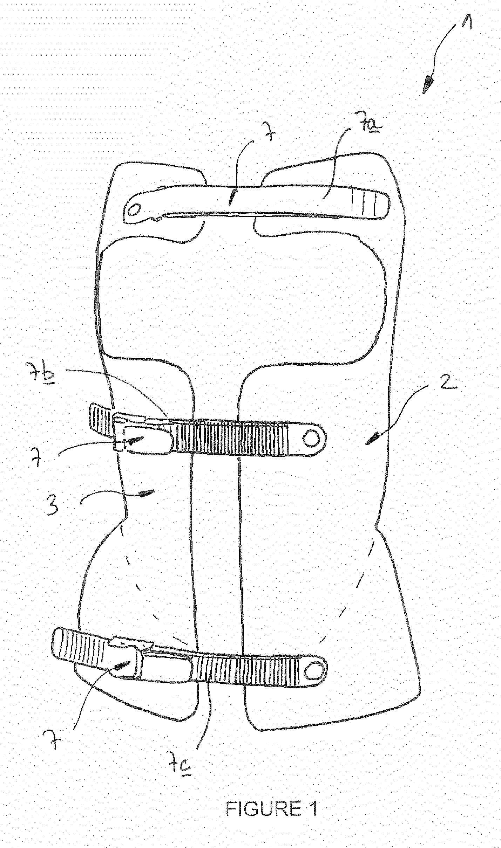

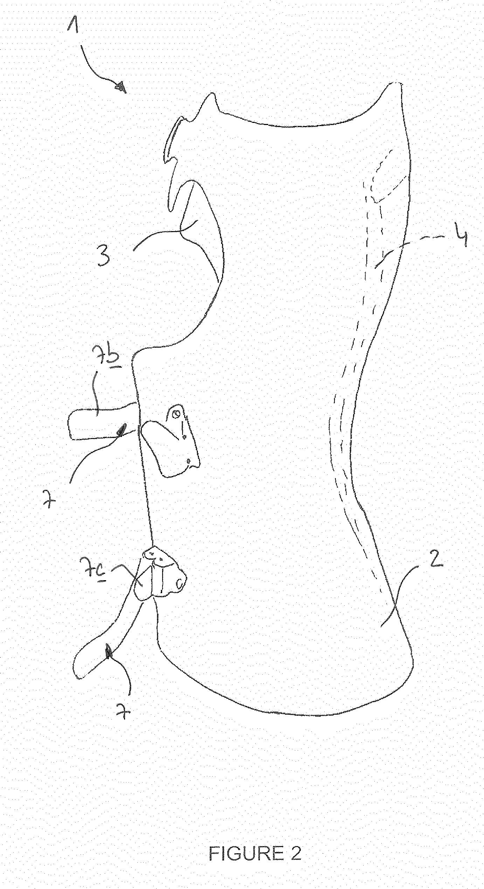

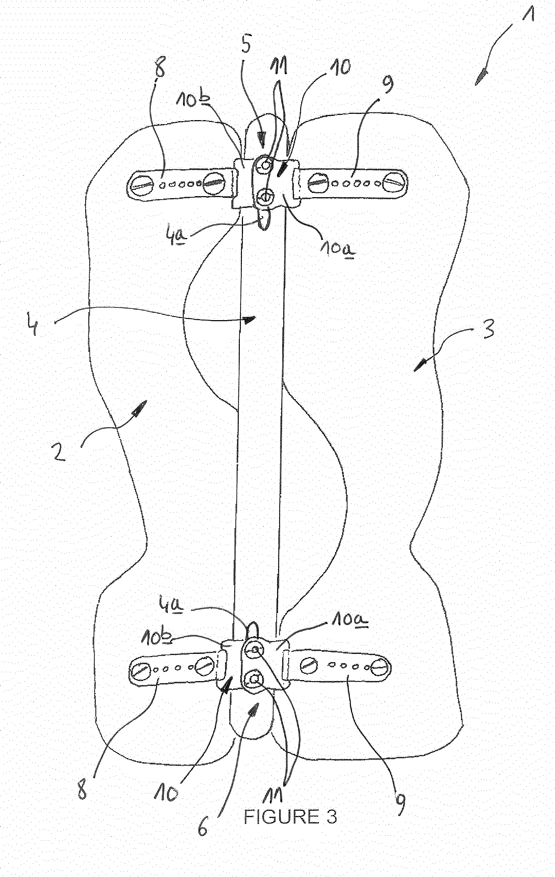

On a montré en figures 1 à 3 un corset rigide asymétrique 1 à valves latérales 2, 3 pour la correction de la scoliose suivant la présente invention.

Ce corset rigide 1 est obtenu à partir de la reconstitution d'un moulage électronique du tronc entier qui est constitué de trois moulages électroniques d'acquisitions instantanées de chaque partie du tronc du patient avec une correction segmentaire.
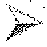 - **Premier moulage électronique du tronc avec une correction selon l'axe vertical en auto-élongation axiale active**
   Pour expliquer l'auto-élongation axiale active, on peut demander au patient « de se grandir » et « d'essayer de toucher le plafond avec sa tête ». Le patient peut s'aider en élevant au maximum les mains le long des supports verticaux. On vérifie sur le monitoring de face l'alignement de l'axe occipital et sur le monitoring de profil l'harmonie entre lordose et cyphose. Le défaut le plus fréquent est l'hyperlordose et l'antéversion du bassin. On explique dans ce cas au patient comment basculer son bassin en avant et en arrière. La priorité est la version du bassin.
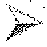 - **Second moulage électronique du tronc avec une correction du segment lombaire en translation frontale et lordose physiologique**
   Pour expliquer la translation frontale ou « shift », on peut demander au patient « d'imaginer qu'il est à la corrida et que le taureau passe avec sa corne vers la pli de taille du côté de la convexité de la courbure lombaire ». On vérifie sur le monitoring de face le pli de la taille et l'alignement sur une même verticale du creux axillaire et du trochanter du côté de la concavité de la courbure lombaire. Les ceintures scapulaires et pelviennes doivent se superposer. On vérifie sur le monitoring de profil la lordose qui doit être physiologique. Les deux mains reposent sur les supports verticaux à la hauteur des épaules.
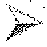 - **Troisième moulage électronique du tronc avec une correction du segment thoracique en inflexion frontale avec tête axée et cyphose physiologique**
   Pour expliquer l'inflexion frontale, on peut demander au patient de réaliser un bending correcteur de la courbure thoracique. Il va alors basculer la ceinture scapulaire et incliner la tête du côté de la convexité. Pour faciliter le bending, la main concave est placée sur la tête avec dans un premier temps le coude dans le plan frontal. On demande alors au patient de réaliser une translation dans le plan frontal de telle sorte que la tête se recentre sur un axe vertical passant par le sacrum (tout en maintenant l'inflexion latérale). On demande enfin au patient de cyphoser le rachis thoracique en lui suggérant par exemple d'imaginer « qu'il prend entre les bras un gros nounours ». On vérifie sur le monitoring de profil l'harmonie de cette cyphose en stimulant le patient avec les mains, par exemple en plaçant un doigt au niveau de l'appendice xiphoïde pour l'inciter à se « casser en deux ». Pour faciliter la cyphose, on peut demander au patient de placer le coude du membre supérieur concave en avant.

Ensuite, on opère la reconstitution du positif représentant le tronc entier du patient au moyen d'un logiciel informatique à superposition des formes obtenue par les moulages électroniques décrits précédemment, successivement pour le socle pelvien symétrique, pour le segment lombaire, pour le segment thoracique et l'épaule située dans la concavité de la scoliose thoracique, pour l'épaule située du côté de la convexité de la scoliose thoracique.

On réalise ensuite par lissage un gabarit en hélicoïde cerclée à cercle générateur horizontal de manière que la forme externe du corset reproduise une colonne torse inverse de la scoliose initiale à partir des corrections segmentaires réelles.

Il en résulte dans la forme du gabarit :
- un espace au niveau de la concavité thoracique se remplissant lors de l'inspiration thoracique
- et un espace au niveau de la concavité abdominale ne restreignant pas la respiration **abdominale.**

L'étape suivante est d du positif par fraiseuse numérique 3D.

La dernière étape est le thermoformage de deux coques hémivalves droite et gauche en polycarbonate. Le thermoformage est réalisé en un seul bloc sur le positif lissé.

Le corset rigide 1 asymétrique à valves latérales ainsi obtenu est constitué de deux coques hémivalves latérales en polycarbonate dont une hémi-coque droite 2 et une hémi-coque gauche 3 à ouverture antérieure.

Le corset rigide 1 comporte dans sa partie dorsale un montant ou mât postérieur 4 galbé en cypho-lordose permettant par l'intermédiaire de charnières réglables supérieure 5 et inférieure 6 la fixation des hémi-coques droite 2 et gauche 3 entre elles. Les charnières 5 et 6 sont prévues réglables dans le plan frontal et placées au niveau des points de tangence d'un fil à plomb sur le deux zones verticales du corset 1.

Chaque charnière 5 et 6 est constituée de deux platines 8 et 9 vissées dans chacune des hémi-coques droite 2 et gauche 3 et reliées entre elles par une liaison verrou 10 immobilisée sur le mât postérieur 4.

Les platines 8 et 9 comportent respectivement à chaque extrémité se faisant face une partie femelle 10**a** et une partie male 10**b** de la liaison verrou 10.La partie femelle 10**a** et la partie male 10**b** de la liaison verrou 10 sont libres en rotation à chaque extrémité des platines 8 et 9 afin de venir en position de fermeture au niveau de lumières oblongues 4**a** aménagées dans le mât postérieur 4. La partie femelle 10**a** et la partie male 10**b** de la liaison verrou 10 de chaque charnière 5 et 6 sont immobilisées entre elles et sur le mât postérieur 4 par l'intermédiaire de vis de serrage 11.

Le corset rigide 1 comporte dans sa partie ventrale trois fermetures antérieures 7 constituées d'une part d'une sangle 7**a** de type « velcro » pour la fermeture supérieure facilitant les mouvements de rotation de la ceinture scapulaire et d'autre part de deux sangles à cliquet 7**b** et 7**c** en plastique semi rigide pour les fermetures centrales et inférieure.

Le corset rigide 1 peut comporter dans sa partie interne des appuis correcteurs complémentaires, non représentés, en fonction de la correction radiologique.

Il doit d'ailleurs être entendu que la description qui précède n'a été donnée qu'à titre d'exemple, et qu'elle ne limite nullement le domaine de l'invention qui est défini par les revendications suivantes.

## Revendications

1. Corset rigide asymétrique à valves latérales pour la correction de la scoliose, comprenant au moins deux coques hémivalves latérales asymétriques ou hémi coques (2, 3) reproduisant une colonne torse en sens inverse de la scoliose, au moins un montant ou mat postérieur (4) galbé en cypho-lordose relié par des charnières postérieures réglables (5, 6) aux desdites coques hémivalves latérales (2, 3) et au moins deux fermetures antérieures (7) positionnées sous la partie ventrale dudit corset.

2. Corset rigide asymétrique à valves latérales pour la correction de la scoliose suivant la revendication 1, **caractérisé en ce que** le socle pelvien est symétrique et de forme quadrangulaire pour favoriser la stabilité sans compression abdominale antérieure ou sus-pubienne.

3. Corset rigide asymétrique à valves latérales pour la correction de la scoliose suivant la revendication 1, **caractérisé en ce que** les charnières postérieures (5, 6) sont réglables dans le plan frontal.

4. Corset rigide asymétrique à valves latérales pour la correction de la scoliose suivant la revendication 1, **caractérisé en ce que** chaque charnière (5, 6) est constituée de deux platines (8, 9) vissées dans chacune des hémi-coques droite (2) et gauche (3) et reliées entre elles par une liaison verrou (10) immobilisée sur le mât postérieur 4.

5. Corset rigide asymétrique à valves latérales pour la correction de la scoliose suivant l'une quelconques des revendications 1 à 4, **caractérisé en ce que** les platines (8, 9) comportent, respectivement à une partie femelle (10**a**) et une partie male (10**b**) de la liaison verrou (10), ladite partie femelle (10**a**) et la partie male (10**b**) étant libre en rotation autour desdites platines (8, 9) afin de venir en position de fermeture au niveau de lumières oblongues (4**a**) aménagées dans le mât postérieur (4), tandis que la partie femelle (10**a**) et la partie male (10**b**) sont immobilisées entre elles et sur le mât postérieur (4) par l'intermédiaire de vis de serrage (11).

6. Corset rigide asymétrique à valves latérales pour la correction de la scoliose suivant la revendication 1, **caractérisé en ce que** l'une au moins des fermetures antérieures (7) est une sangle (7**a**) de type « velcro » assurant la fermeture supérieure du corset (1) et facilitant les mouvements de rotation de la ceinture scapulaire

7. Corset rigide asymétrique à valves latérales pour la correction de la scoliose suivant la revendication 1, **caractérisé en ce que** les autres fermetures antérieures (7) sont des sangles à cliquet (7**b**, 7**c**) en plastique semi rigide pour la fermeture centrale et inférieure.

8. Procédé pour la réalisation du corset rigide asymétrique à valves latérales pour la correction de la scoliose suivant la revendication 1, **caractérisé en ce que** qu'il consiste successivement :
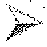 A procéder à un premier moulage électronique du tronc du patient en auto-élongation axiale active,
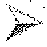 A procéder à un second moulage électronique du tronc du patient en translation lombaire et lordose physiologique,
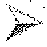 A procéder à un troisième moulage électronique du tronc du patient en inflexion thoracique frontale et cyphose physiologique,
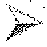 A reconstituer la colonne torse inverse de la scoliose du patient par la superposition des trois moulages électroniques,
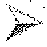 A réaliser le positif du gabarit par fraiseuse numérique 3D,
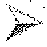 A thermoformer les deux coques hémivalves droite et gauche en polycarbonate en un seul bloc sur le positif lissé.

## Patentansprüche

1. Asymmetrisches steifes Korsett mit seitlichen Ventilen zur Korrektur der Skoliose, umfassend mindestens zwei asymmetrische seitliche halbschalenförmige Schalen oder Halbschalen (2, 3), die eine Torsosäule in umgekehrter Richtung der Skoliose reproduzieren, mindestens einen kypholordosisch geschwungener Pfosten oder hinteren Mast (4), der anhand von regulierbaren hinteren Scharnieren (5, 6) mit den seitlichen halbschalenförmigen Schalen (2, 3) verbunden ist, und mindestens zwei vordere Verschlüsse (7), die unter dem Bauchteil des Korsetts positioniert sind.

2. Asymmetrisches steifes Korsett mit seitlichen Ventilen zur Korrektur der Skoliose nach Anspruch 1, **dadurch gekennzeichnet, dass** der Beckenboden symmetrisch und viereckig ist, um die Stabilität ohne abdominale Kompression vorn oder suprapubisch zu unterstützen.

3. Asymmetrisches steifes Korsett mit seitlichen Ventilen zur Korrektur der Skoliose nach Anspruch 1, **dadurch gekennzeichnet, dass** die hinteren Scharniere (5, 6) in frontaler Ebene einstellbar sind.

4. Asymmetrisches steifes Korsett mit seitlichen Ventilen zur Korrektur der Skoliose nach Anspruch 1, **dadurch gekennzeichnet, dass** jedes Scharnier (5, 6) aus zwei Platten (8, 9) besteht, die in jeder der Halbschalen rechts (2) und links (3) verschraubt und miteinander durch eine Riegelverbindung (10) verbunden sind, die auf dem hinteren Mast (4) fixiert ist.

5. Asymmetrisches steifes Korsett mit seitlichen Ventilen zur Korrektur der Skoliose nach einem der Ansprüche 1 bis 4, **dadurch gekennzeichnet, dass** die Platten (8, 9) jeweils einen weiblichen Teil (10a) und einen männlichen Teil (10b) der Riegelverbindung (10) aufweisen, wobei der weibliche Teil (10a) und der männliche Teil (10b) um die Platten (8, 9) rotatorisch frei sind, um in Verschlussposition im Bereich von Langlöchern (4a) zu kommen, die im hinteren Mast (4) eingerichtet sind, wohingegen der weibliche Teil (10a) und der männliche Teil (10b) aneinander und auf dem hinteren Mast (4) anhand von Spannschrauben (11) fixiert sind.

6. Asymmetrisches steifes Korsett mit seitlichen Ventilen zur Korrektur der Skoliose nach Anspruch 1, **dadurch gekennzeichnet, dass** mindestens einer der vorderen Verschlüsse (7) ein Gurt (7a) vom Typ "Velcro" ist, welcher den oberen Verschluss des Korsetts (1) sichert und die Rotationsbewegungen des Schultergürtels erleichtert.

7. Asymmetrisches steifes Korsett mit seitlichen Ventilen zur Korrektur der Skoliose nach Anspruch 1, **dadurch gekennzeichnet, dass** die anderen vorderen Verschlüsse (7) Klinkengurte (7b, 7c) aus halbsteifem Kunststoff für den Verschluss in der Mitte und unten sind.

8. Verfahren zur Herstellung des asymmetrischen steifen Korsetts mit seitlichen Ventilen zur Korrektur der Skoliose nach Anspruch 1, **dadurch gekennzeichnet, dass** es aufeinanderfolgende besteht aus:
- Durchführen einer ersten elektronischen Abformung des Rumpfs des Patienten in aktiver axialer Selbstdehnung,
- Durchführen einer zweiten elektronischen Abformung des Rumpfs des Patienten in lumbaler Translation und physiologischer Lordose,
- Durchführen einer dritten elektronischen Abformung des Rumpfs des Patienten in frontaler Thoraxbeugung und physiologischer Kyphose,
- Nachbildung der inversen Torsosäule der Skoliose des Patienten durch Übereinanderlagern der drei elektronischen Abformungen,
- Herstellen des Positivs der Schablone durch digitales 3D-Fräsen,
- thermisches Formen der zwei halbschalenförmigen Schalen rechts und links aus Polycarbonat in einem einzigen Block auf dem geglätteten Positiv.

## Claims

1. Asymmetric rigid corset with lateral valves for correcting scoliosis, comprising at least two asymmetric lateral half-valve casings or half-casings (2, 3) reproducing a column that is twisted in the opposite direction to the scoliosis, at least one posterior mast or upright (4) with kypholordosis curvature connected by adjustable posterior hinges (5, 6) to said lateral half-valve casings (2, 3) and at least two anterior closures (7) positioned beneath the ventral portion of said corset.

2. Asymmetric rigid corset with lateral valves for correcting scoliosis according to claim 1, **characterised in that** the pelvic base is symmetric and has a quadrangular shape so as to facilitate stability without anterior abdominal or sub-pubic compression.

3. Asymmetric rigid corset with lateral valves for correcting scoliosis according to claim 1, **characterised in that** the posterior hinges (5, 6) can be adjusted in the frontal plane.

4. Asymmetric rigid corset with lateral valves for correcting scoliosis according to claim 1, **characterised in that** each hinge (5, 6) is formed by two plates (8, 9) screwed into each of the right (2) and left (3) half-casings and connected to one another by a locking link (10) immobilised on the posterior mast (4).

5. Asymmetric rigid corset with lateral valves for correcting scoliosis according to any one of claims 1 to 4, **characterised in that** the plates (8, 9) respectively include a female portion (10a) and a male portion (10b) of the locking link (10), said female portion (10a) and the male portion (10b) being free to rotate about said plates (8, 9) in order to reach a closing position at oblong slots (4a) made in the posterior mast (4), whereas the female portion (10a) and the male portion (10b) are immobilised relative to one another and on the posterior mast (4) by means of an attachment screw (11).

6. Asymmetric rigid corset with lateral valves for correcting scoliosis according to claim 1, **characterised in that** at least one of the anterior closures (7) is a strap (7a) of the "Velcro" type, procuring the upper closing of the corset (1) and facilitating the rotational movements of the scapular girdle.

7. Asymmetric rigid corset with lateral valves for correcting scoliosis according to claim 1, **characterised in that** the other anterior closures (7) are ratchet straps (7b, 7c) made of semi-rigid plastic for the central and lower closures.

8. Method for producing the asymmetric rigid corset with lateral valves for correcting scoliosis according to claim 1, **characterised in that** it successively consists of:
- carrying out a first electronic moulding of the patient's trunk in active axial self-elongation,
- carrying out a second electronic moulding of the patient's trunk in lumbar translation and physiological lordosis,
- carrying out a third electronic moulding of the patient's trunk in frontal thoracic inflexion and physiological kyphosis,
- reconstituting the column twisted in the opposite direction to the patient's scoliosis by superimposing the three electronic mouldings,
- producing the positive of the template by 3D digital milling,
- thermoforming the two right and left half-valve casings out of polycarbonate in one piece on the smoothed positive.
